# FASCICULE DE BREVET EUROPEEN

(11) **EP 0 775 483 B1**
(45) Date de publication et mention de la délivrance du brevet: **08.05.2002**
(21) Numéro de dépôt: 96402279.2
(22) Date de dépôt: 25.10.1996
(51) Int. Cl.: A61K 7/48, A61K 7/025, A61K 7/027, A61K 7/032

(54) **Utilisation d'une composition comprenant un système polymérique**
Verwendung einer ein Polymersystem enthaltenden Zubereitung
Use of a composition containing a polymer system

(30) Priorité: 27.10.1995 FR 9512833; 14.02.1996 FR 9601811
(43) Date de publication de la demande: 28.05.1997
(73) Titulaire: L'OREAL, 75008 Paris (FR)
(72) Inventeur: De la Poterie, Valérie, 77820 Le Chatelet en Brie (FR); Bara, Isabelle, 75013 Paris (FR)
(74) Mandataire: Dodin, Catherine

(56) Documents cités:
- EP-A- 0 140 325
- EP-A- 0 206 671
- EP-A- 0 530 084
- EP-A- 0 566 442
- EP-A- 0 581 581
- EP-A- 0 627 212
- EP-A- 0 628 304
- EP-A- 0 636 361
- EP-A- 0 637 600
- EP-A- 0 648 485
- EP-A- 0 655 234
- EP-A- 0 658 609
- EP-A- 0 687 462
- EP-A- 0 820 764
- WO-A-91/12793
- FR-A- 2 718 350
- GB-A- 2 238 242
- US-A- 4 988 502
- PATENT ABSTRACTS OF JAPAN vol. 10, no. 383 (C-393) [2440] , 23 Décembre 1986 & JP-A-61 176512 (KYOKO WATANABE)
- PATENT ABSTRACTS OF JAPAN vol. 13, no. 308 (C-617) [3656] , 14 Juillet 1989 & JP-A-01 096110 (SHIEIDO CO)
- CHEMICAL ABSTRACTS, vol. 89, no. 22, 27 Novembre 1978 Columbus, Ohio, US; abstract no. 185915, page 363; colonne r; XP002004752 & JP-A-07 894 041 (KOBAYASHI KOSE CO LTD.)
- CHEMICAL ABSTRACTS, vol. 97, no. 18, 1 Novembre 1982 Columbus, Ohio, US; abstract no. 150584, page 382; colonne r; XP002004753 & JP-A-08 263 315 (TEIJIN LTD.)

## Description

La présente invention a trait à une composition cosmétique de maquillage des lèvres. Ladite composition comprend en particulier une dispersion aqueuse de particules de polymère filmogène et peut être utilisée en tant que produit de maquillage des lèvres.

Les compositions à appliquer sur la peau, les semi-muqueuses et/ou les muqueuses telles que les rouges à lèvres et les fonds de teint, se présentent généralement sous forme de stick, de pâte souple ou de pâte coulée, et comprennent des corps gras tels que des huiles, des composés pâteux et/ou des cires, et une phase particulaire généralement composée de charges et de pigments.

Ces compositions, lorsqu'elles sont appliquées sur la peau, les muqueuses ou les semi-muqueuses, présentent toutefois l'inconvénient de transférer. On entend par là que la composition est susceptible de se déposer, au moins en partie, sur certains supports avec lesquels elle est mise en contact, tels que, par exemple, un verre, une tasse, un vêtement ou la peau. En se déposant, ladite composition laisse une trace sur ledit support. Il s'en suit donc une persistance médiocre de la composition sur la peau, les semi-muqueuses ou les muqueuses, et la nécessité de renouveler régulièrement son application.
Par ailleurs, l'apparition de traces inacceptables sur certains vêtements et notamment sur les cols de chemisier peut écarter certaines femmes de l'utilisation de ce type de maquillage.
Un autre inconvénient de ces compositions réside dans le problème de migration. On a en effet constaté que certaines compositions avaient tendance à se propager à l'intérieur des ridules qui entourent les lèvres, dans le cas des rouges à lèvres.Tous ces phénomènes engendrent un effet inesthétique que l'on souhaite bien évidemment éviter.

Depuis plusieurs années, de nombreux cosméticiens se sont intéressés aux compositions cosmétiques, notamment de rouge à lèvres ou de fond de teint 'sans transfert'. Ainsi, il a été envisagé des compositions de rouge à lèvres 'sans transfert' contenant de 1 à 70% en poids de résine liquide de silicone à motifs répétitifs silicates, de 10 à 98% en poids d'une huile de silicone volatile et des charges pulvérulentes. Toutefois, le film obtenu sur les lèvres après évaporation de l'huile de silicone présente l'inconvénient de devenir inconfortable au cours du temps (sensation de dessèchement et de tiraillement).
On connaît également des rouges à lèvres 'sans transfert' contenant une silicone volatile et une résine de silicone comportant une chaîne estérifiée pendante ayant au moins 12 atomes de carbone. Le film de rouge à lèvres présente notamment l'inconvénient de manquer de confort à l'application, en particulier d'être trop sec.
Ainsi, d'une manière générale, l'association d'huiles volatiles avec certains composés siliconés permet d'obtenir un résultat 'sans transfert' satisfaisant. Toutefois, les films obtenus après application de ces compositions et évaporation des volatils présentent néanmoins l'inconvénient d'être relativement mats, et conduisent ainsi à un maquillage peu brillant.

Il subsiste donc le besoin d'une composition cosmétique qui transfère peu ou pas du tout, c'est-à-dire d'une composition 'sans transfert', tout en possédant de bonnes propriétés cosmétiques, et en particulier permettant l'obtention d'un film qui peut être, au choix, plus ou moins brillant.
La présente invention a pour but de proposer une composition qui permet d'obtenir un film de très bonne tenue, qui ne transfère pas et ne tache pas un support avec lequel il serait en contact, et qui ne migre pas au cours du temps, tout en permettant d'obtenir un maquillage et/ou un film brillant.

Ainsi, un objet de l'invention est l'utilisation cosmétique dans une composition de maquillage des lèvres d'un système polymérique tel que défini dans les revendications.

Certes, il est connu d'utiliser des polymères dans des compositions à application topique et notamment des compositions à destinées thérapeutiques contenant une dispersion aqueuse de polymère (voir EP-A-581581) ; des mascaras contenant des dispersions aqueuses de polymères (EP-A-655234, EP-A-628304, EP-A-636361, EP-A-2238242), des mascaras contenant des polymères hydrosolubles (voir WO-A-91/12793, US-a-4988502, EP-A-530084) ; des compositions pour les lèvres anhydres contenant de la poudre d'un copolymère de polyvilylidène (EP-A-566442) ; des vernis à ongles contenant des dispersions aqueuses de polymère (EP-A-637600) ; des poudres compactes visage contenant des polymères hydrosolubles (EP-A-140325, JP-A-0196110) et des compositions cosmétiques contenant des résines (abrégé 185917 f. Chemical Abstracts vol 89-n°22- 27/11/78) et des eyeliners contenant une dispersion aqueuse de polymère. Toutefois, ces documents ne décrivent pas de composition contenant une dispersion de polymère filmogène conduisant à un film souple, élastique tout en étant résistant et sans transfert, suivant le mouvement des lèvres, ayant une élongation d'au moins 300 %.

On a constaté que la composition selon l'utilisation de l'invention est facilement applicable et s'étale aisément et uniformément sur les lèvres du visage.
La composition à laquelle s'applique l'invention trouve notamment une application particulièrement intéressante dans le domaine du soin et/ou du maquillage des semi-muqueuses.

Parmi les semi-muqueuses, on entend plus particulièrement les lèvres du visage.

La composition à laquelle s'applique l'invention permet l'obtention d'un film homogène, qui présente une texture légère et reste confortable à porter tout au long de la journée. Le film n'est pas du tout collant, tout en étant mou, souple, élastique et flexible sur la peau; il suit les mouvements des lèvres sans se craqueler et/ou se décoller. Il adhère parfaitement sur les lèvres du visage.
La composition trouve donc une application toute particulière en tant que composition à appliquer sur les lèvres, notamment en tant que rouge à lèvres.
D'autre part, le film obtenu peut être très brillant, ou plus ou moins mat, selon la nature des constituants de la composition, d'où une gamme plus étendue de produits de maquillage, brillants ou mats, au choix.

La composition à laquelle s'applique l'invention comprend donc un système polymérique qui comprend au moins une dispersion aqueuse de particules de polymère filmogène.

Parmi les polymères filmogènes utilisables dans le cadre de la présente invention, on peut citer les polymères synthétiques, de type polycondensat ou de type radicalaires, et leurs mélanges.
On peut ainsi citer, parmi les polycondensats, les polyuréthannes anioniques, cationiques, non ioniques ou amphotères, les polyuréthannes-acryliques, les polyuréthannes-polyvinylpirrolidones, les polyester-polyuréthannes, les polyéther-polyuréthannes, les polyurées, les polyurée/polyuréthannes, et leurs mélanges.
Le polyuréthanne peut être, par exemple, un copolymère polyuréthanne, polyurée/uréthanne ou polyurée, aliphatique, cycloaliphatique ou aromatique, comportant, seule ou en mélange,
- au moins une séquence d'origine polyester aliphatique linéaire ou ramifié et/ou cycloaliphatique et/ou aromatique, et/ou
- au moins une séquence d'origine polyéther aliphatique et/ou cycloaliphatique et/ou aromatique, et/ou
- au moins une séquence siliconée, substituée ou non, ramifiée ou non, par exemple polydiméthylsiloxane ou polyméthylphénylsiloxane, et/ou
- au moins une séquence comportant des groupes fluorés.
Les polyuréthannes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comp ortant en plus soit un groupement acide carboxylique ou carboxylate, soit un groupement acide sulfonique ou sulfonate, soit encore un groupement amine tertiaire neutralisable ou un groupement ammonium quaternaire.

On peut également citer les polyesters, les polyesters amides, les polyesters à chaîne grasse, les polyamides, et les résines époxyesters.
Les polyesters peuvent être obtenus, de façon connue, par polycondensation de diacides aliphatiques ou aromatiques avec des diols aliphatiques ou aromatiques ou des polyols. Comme diacides aliphatiques, on peut utiliser l'acide succinique, l'acide glutarique, l'acide adipique, l'acide pimélique, l'acide subérique ou l'acide sébacique. Comme diacides aromatiques, on peut utiliser l'acide téréphtalique ou l'acide isophtalique, ou bien encore un dérivé tel que l'anhydride phtalique. Comme diols aliphatiques, on peut utiliser l'éthylène glycol, le propylène glycol, le diéthylène glycol, le néopentyl glycol, le cyclohexane diméthanol, le 4,4'-(1-méthylpropylidène)bisphénol. Comme polyols, on peut utiliser le glycérol, le pentaérythritol, le sorbitol, le triméthylol propane.
Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.
Comme monomère porteur de groupement anionique pouvant être utilisé lors de la polycondensation, on peut citer par exemple l'acide diméthylol propionique, l'acide trimellitique ou un dérivé tel que l'anhydride trimellitique, le sel de sodium de l'acide sulfo-3 pentanediol, le sel de sodium de l'acide 5-sulfo 1,3-benzène dicarboxylique .
Les polyesters à chaîne grasse peuvent être obtenus par l'utilisation de diols à chaîne grasse lors de la polycondensation.
Les résines époxyesters peuvent être obtenues par polycondensation d'acides gras avec un condensat aux extrémités α, ω - diépoxy.
Les polymères de type radicalaires peuvent être notamment des polymères, ou des copolymères, acryliques et/ou vinyliques. On utilise de préférence des polymères radicalaires anioniques.
Comme monomère porteur de groupement anionique pouvant être utilisé lors de la polymérisation radicalaire, on peut citer l'acide acrylique, l'acide méthacrylique, l'acide crotonique, l'anhydride maléique, l'acide acrylamido-2 méthyl-2 propane sulfonique.
Les polymères acryliques peuvent résulter de la copolymérisation de monomères choisis parmi les esters et/ou les amides de l'acide acrylique ou de l'acide méthacrylique. Comme exemple de monomères de type ester, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle. Comme exemple de monomères de type amide, on peut citer le N-t-butyl acrylamide et le N-t-octyl acrylamide.
On utilise de préférence des polymères acryliques obtenus par copolymérisation de monomères à insaturation éthylénique contenant des groupements hydrophiles, de préférence de nature non ionique, tels que l'acrylate d'hydroxyéthyle, l'acrylate de 2-hydroxypropyle, le méthacrylate d'hydroxyéthyle, le méthacrylate de 2-hydroxypropyle.
Les polymères vinyliques peuvent résulter de l'homopolymérisation ou de la copolymérisation de monomères choisis parmi les esters vinyliques, le styrène ou le butadiène. Comme exemple d'esters vinyliques, on peut citer l'acétate de vinyle, le néodécanoate de vinyle, le pivalate de vinyle, le benzoate de vinyle et le t-butyl benzoate de vinyle.
On peut également utiliser des copolymères acryliques/silicones, ou encore des copolymères nitrocellulose/acryliques.
Les polymères d'origine naturelle, éventuellement modifiés, peuvent être choisis parmi la résine shellac, la gomme de sandaraque, les dammars, les élémis, les copals, les dérivés cellulosiques, et leurs mélanges.
On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

La dispersion aqueuse comprenant un ou plusieurs polymères filmogènes peut être préparée par l'homme du métier sur base de ses connaissances générales.
Afin d'améliorer le caractère filmogène d'un polymère, par exemple en abaissant sa température de transition vitreuse, il est possible d'ajouter à la dispersion un agent de coalescence, qui peut être choisi parmi les agents de coalescence connus. Dans la présente description, on entend par 'dispersion de polymère filmogène', une dispersion susceptible de former un film, comprenant ou ne comprenant pas d'agent de coalescence.
La teneur en matière sèche desdites dispersions aqueuses selon la présente invention peut être de l'ordre de 5-60% en poids, et de préférence 30-40%.
La composition peut comprendre 1-60% en poids, de préférence 5-40% en poids de matière sèche de polymères filmogènes.
La taille des particules de polymères en dispersion aqueuse peut être comprise entre 10-500 nm, et est de préférence comprise entre 20 et 150 nm, ce qui permet d'obtenir un film ayant une brillance remarquable.

Afin de réaliser la présente invention, il est nécessaire que le système polymérique permette l'obtention d'un film sur le support sur lequel il est déposé, ledit film ayant une élongation supérieure à 300%.
A cette fin, ledit système polymérique comprend une dispersion aqueuse de particules de polymère filmogène. Lorsque ladite dispersion de particules de polymère ne permet pas d'obtenir, seule, un film ayant une élongation supérieure à 300%, il est possible d'ajouter un composé dont la fonction est de modifier l'élongation du film, de la manière souhaitée. Un tel composé sera appelé dans la suite de la présente description, 'agent plastifiant'. Le système polymérique comprend alors la dispersion de particules de polymère filmogène et l'agent plastifiant.

Ledit agent plastifiant peut être choisi parmi tous les composés connus de l'homme du métier comme étant susceptibles de remplir la fonction recherchée. Cet agent peut être hydrosoluble ou insoluble dans l'eau et peut éventuellement se présenter sous forme de dispersion aqueuse.
En particulier, on peut citer, seuls ou en mélange, les plastifiants usuels, tels que:
- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther;
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- des esters d'acides notamment carboxyliques tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; les huiles de silicone,
- des polymères hydrosolubles ou en dispersion aqueuse, ayant une température de transition vitreuse faible, inférieure à 25°C, de préférence inférieure à 15°C.
La quantité d'agent plastifiant est choisie par l'homme du métier sur base de ses connaissances générales, de manière à obtenir un système polymérique conduisant à un film ayant une élongation supérieure à 300%, tout en conservant à la composition des propriétés cosmétiquement acceptables.

La composition selon l'invention comprend donc un système polymérique qui comprend une dispersion aqueuse de particules de polymère filmogène, ledit système permettant l'obtention d'un film ayant une élongation supérieure à 300%. Elle est mesurée selon la norme ASTM Standards, volume 06.01 D 2370-92 'Standard Test Method for Tensile Properties of Organic Coatings'.

La composition peut en outre comprendre au moins un colorant hydrosoluble et/ou au moins un pigment, utilisés de manière usuelle dans le domaine de la cosmétique et du maquillage.

Par pigments, il faut comprendre des particules blanches ou colorées, minérales ou organiques, insolubles dans le milieu, destinées à colorer et/ou opacifier la composition. Les pigments peuvent être présents dans la composition à raison de 0-20% en poids de la composition finale, et de préférence à raison de 1-5%. Ils peuvent être blancs ou colorés, minéraux et/ou organiques, de taille usuelle ou nanométrique. On peut citer, parmi les pigments et/ou nanopigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.
Parmi les colorants hydrosolubles, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, leurs mélanges.

On peut également ajouter dans la composition selon l'invention tout additif connu tel que des agents épaississants, par exemple des argiles, des gommes, des silices, les dérivés cellulosiques, un polymère synthétique tel qu'un polymère acrylique ou un polymère associatif de type polyuréthanne; une gomme naturelle telle que la gomme xanthane; des agents d'étalement; des dispersants; des conservateurs; des agents antimousses; des agents mouillants; des filtres UV; des parfums; des charges; des actifs cosmétiques ou pharmaceutiques; des hydratants; des vitamines et leurs dérivés; des matières biologiques et leurs dérivés.
Bien entendu l'homme du métier veillera à choisir ce ou ces éventuels additifs et/ou leur quantité, de manière telle que les propriétés avantageuses de la composition selon l'invention ne soient pas, ou substantiellement pas, altérées par l'adjonction envisagée.

Le pH de la composition finale obtenue est de préférence inférieur à 9. Cette composition doit bien entendu être apte à se déposer sur un support tel que les semi-muqueuses.
La composition selon l'invention peut se présenter sous forme fluide, gélifiée, semi-solide, pâte souple, voire solide telle que de stick ou bâton.
Elle trouve en particulier une application en tant que produit de maquillage, notamment en tant que rouge à lèvres.

L'invention est illustrée plus en détail dans les exemples suivants.

### Exemple 1

On prépare des dispersions aqueuses de différents polymères filmogènes, lesdites dispersions permettant l'obtention de films ayant des élongations variées.
On dépose la composition sur un support de manière à former un film.
On mesure l'élongation du film obtenu et l'on apprécie la tenue du film sur les lèvres.

On obtient les résultats suivants :

| Polymère | Elongation | Appréciation visuelle |
|---|---|---|
| Polyuréthanne 1 SANCURE 2060 | 120 | craquèle très vite au milieu des lèvres; se décolle vite sur les côtés |
| Polyuréthanne 2 SANCURE 815 | 200 | craquèle un peu; se décolle après quelque temps |
| Polyuréthanne 3 NEOREZ R-974 | 280 | craquèle un peu; se décolle après quelque temps |
| Polyuréthanne 4 NEOREZ R-981 | 330 | long à craqueler; ne se décolle pas |
| Polyuréthanne 5 SANCURE 878 | 425 | très long à craqueler; ne se décolle pas |
| Polyuréthanne 6 SANCURE 2255 | 550 | très long à craqueler; ne se décolle pas |
| Polyuréthanne 7 SANCURE 861 | 580 | ne se craquèle pas; très souple |

On constate donc que l'on obtient un film adéquat, ayant une bonne tenue et relativement souple, lorsque le polymère permet l'obtention d'un film ayant une élongation supérieure à 300%.

### Exemple 2

On prépare un rouge à lèvres ayant la composition suivante :
. dispersion aqueuse de polyuréthanne (élongation 330%) 95 g
. pigment 1 g
. agent plastifiant (glycérine) 1,25 g

On obtient une composition facile à appliquer sur les lèvres; le film obtenu est brillant; il ne transfère pas et ne migre pas dans les ridules; il résiste bien et suit le mouvement des lèvres.

### Exemple 3 (contre-exemple)

On prépare un rouge à lèvres ayant la composition suivante :
. dispersion aqueuse de polyuréthanne (élongation 120%) 95 g
. pigment 1 g
. agent plastifiant (glycérine) 1,25 g

On obtient un film qui craquèle très rapidement après son application sur les lèvres.

## Revendications

1. Utilisation cosmétique dans une composition de maquillage des lèvres du visage, d'un système polymérique comprenant une dispersion aqueuse de particules de polymère filmogène synthétique, les particules ayant une taille comprise entre 10 et 500nm, ledit système permettant l'obtention d'un film sur les lèvres, ayant une élongation supérieure à 300%, suivant les mouvements des lèvres, et étant de très bonne tenue et/ou qui ne transfère pas et/ou qui ne migre pas et/ou qui ne tache pas.

2. Utilisation selon la revendication précédente, afin d'obtenir un film souple et/ou élastique et/ou flexible et/ou un film qui ne se craquèle pas et/ou ne se décolle pas.

3. Utilisation selon l'une des revendications précédentes, afin d'obtenir un film brillant.

4. Utilisation selon l'une des revendications précédentes, dans laquelle le polymère filmogène est choisi parmi les polyuréthannes anioniques, cationiques, non ioniques ou amphotères; les polyuréthannes-acryliques, les polyuréthannes-polyvinylpirrolidones, les polyester-polyuréthannes, les polyéther-polyuréthannes, les polyurées, les polyurée/polyuréthannes; les polyesters, les polyesters amides, les polyesters à chaîne grasse, les polyamides, les résines époxyesters; les polymères et/ou copolymères, acryliques et/ou vinyliques; les copolymères acryliques/silicones; les copolymères nitrocellulose/acryliques; les polymères d'origine naturelle, éventuellement modifiés; les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes; et leurs mélanges.

5. Utilisation selon l'une des revendications précédentes, dans laquelle la taille des particules de polymères en dispersion aqueuse est comprise entre 20 et 150 nm.

6. Utilisation selon l'une des revendications précédentes, dans laquelle le système polymérique comprend en outre un agent plastifiant.

7. Utilisation selon la revendication 6, dans laquelle l'agent plastifiant est choisi parmi les glycols et leurs dérivés; les esters de glycérol; les dérivés de propylène glycol ; des esters d'acides notamment carboxyliques; des dérivés oxyéthylénés; des polymères hydrosolubles ou en dispersion aqueuse, ayant une température de transition vitreuse faible, inférieure à 25°C, de préférence inférieure à 15°C; et leurs mélanges.

8. Utilisation selon l'une des revendications précédentes, dans laquelle la composition comprend en outre au moins un colorant hydrosoluble et/ou au moins un pigment.

9. Utilisation selon l'une des revendications précédentes, dans laquelle la composition est sous forme d'un rouge à lèvres.

## Patentansprüche

1. Kosmetische Verwendung eines Polymersystems, das eine wäßrige Dispersion von Partikeln eines synthetischen filmbildenden Polymers enthält, in einer Zusammensetzung zum Schminken der Lippen, wobei die Partikel eine Größe von 10 bis 500 nm aufweisen und wobei mit der Zusammensetzung auf den Lippen ein Film gebildet werden kann, der eine Dehnung über 300 % aufweist, den Bewegungen der Lippen folgt, sehr gut haftet und/oder sich nicht überträgt und/oder keine Migration zeigt und/oder keine Flecken verursacht.

2. Verwendung nach dem vorhergehenden Anspruch zur Herstellung eines weichen und/oder elastischen und/oder flexiblen Films und/oder eines Films, der keine Risse bildet und/oder sich nicht abhebt.

3. Verwendung nach einem der vorhergehenden Ansprüche zur Herstellung eines glänzenden Films.

4. Verwendung nach einem der vorhergehenden Ansprüche, wobei das filmbildende Polymer unter den anionischen, kationischen, nichtionischen oder amphoteren Polyurethanen, Polyurethan-Acryl-Polymeren, Polyurethan-Polyvinylpyrrolidonen, Polyester-Polyurethanen, Polyether-Polyurethanen, Polyharnstoffen, Polyharnstoff/Polyurethanen, Polyestern, Polyesteramiden, Polyestern mit Fettkette, Polyamiden, Epoxyesterharzen, Acryl- und/oder Vinylpolymeren, Acryl- und/oder Vinylcopolymeren, Acryl/Siliconcopolymeren, Nitrocellulose/Acrylcopolymeren, Polymeren natürlicher Herkunft, die gegebenenfalls modifiziert sind, Polymeren, die bei der radikalischen Polymerisation eines oder mehrerer radikalischer Monomere im Innern und/oder zum Teil an der Oberfläche von vorab gebildeten Partikeln mindestens eines Polymers entstehen, das unter den Polyurethanen, Polyharnstoffen, Polyestern, Polyesteramiden und/oder Alkydharzen ausgewählt ist, und deren Gemischen ausgewählt ist.

5. Verwendung nach einem der vorhergehenden Ansprüche, wobei die Partikel des filmbildenden Polymers in wäßriger Dispersion eine Größe im Bereich von 20 bis 150 nm aufweisen.

6. Verwendung nach einem der vorhergehenden Ansprüche, wobei das Polymersystem ferner einen Weichmacher enthält.

7. Verwendung nach Anspruch 6, wobei der Weichmacher unter den Glykolen und ihren Derivaten, Glycerinestern, Propylenglykolderivaten, Estern von Säuren, insbesondere von Carbonsäuren, ethoxylierten Derivaten; wasserlöslichen Polymeren oder Polymeren in wäßriger Dispersion, die eine niedrige Glasübergangstemperatur unter 25 °C und vorzugsweise unter 15 °C aufweisen, und deren Gemischen ausgewählt ist.

8. Verwendung nach einem der vorhergehenden Ansprüche, wobei die Zusammensetzung ferner mindestens ein unlösliches Farbmittel und/oder mindestens ein Pigment enthält.

9. Verwendung nach einem der vorhergehenden Ansprüche, wobei die Zusammensetzung als Lippenstift vorliegt.

## Claims

1. Cosmetic use, in a make-up composition for the lips of the face, of a polymeric system comprising an aqueous dispersion of particles of synthetic film-forming polymer, the particles having a size of between 10 and 500 nm, the said system allowing the obtaining of a film on the lips, having an extension greater than 300%, according to the movements of the lips, and exhibiting very good retention and/or which does not transfer and/or which does not migrate and/or which does not stain.

2. Use according to the preceding claim, for obtaining a supple and/or elastic and/or flexible film and/or a film which does not crack and/or does not come off.

3. Use according to one of the preceding claims, for obtaining a bright film.

4. Use according to one of the preceding claims, in which the film-forming polymer is chosen from anionic, cationic, nonionic or amphoteric polyurethanes; polyurethanes-acrylics, polyurethanes-polyvinylpyrrolidones, polyester-polyurethanes, polyether-polyurethanes, polyureas, polyureas/polyurethanes; polyesters, polyester-amides, fatty chain polyesters, polyamides, epoxy ester resins; acrylic and/or vinyl polymers and/or copolymers; acrylic/silicone copolymers; nitrocellulose/acrylic copolymers; optionally modified polymers of natural origin; polymers resulting from free-radical polymerization of one or more free-radical monomers inside and/or partially at the surface, of preexisting particles of at least one polymer chosen from the group consisting of polyurethanes, polyureas, polyesters, polyester-amides and/or alkyds; and mixtures thereof.

5. Use according to one of the preceding claims, in which the size of the particles of polymers in aqueous dispersion is between 20 and 150 nm.

6. Use according to one of the preceding claims, in which the polymeric system comprises, in addition, a plasticizing agent.

7. Use according to Claim 6, in which the plasticizing agent is chosen from glycols and derivatives thereof; glycerol esters; derivatives of propylene glycol; esters of acids, in particular carboxylic acids; oxyethylenated derivatives; polymers which are water-soluble or in aqueous dispersion, having a low glass transition temperature, of less than 25°C, preferably of less than 15°C; and mixtures thereof.

8. Use according to one of the preceding claims, in which the composition comprises, in addition, at least one water-soluble colorant and/or at least one pigment.

9. Use according to one of the preceding claims, in which the composition is in the form of a lipstick.
